# EUROPEAN PATENT SPECIFICATION

(11) **EP 1 859 751 B1**
(45) Date of publication and mention of the grant of the patent: **15.09.2010**
(21) Application number: 07107495.9
(22) Date of filing: 04.05.2007
(51) Int. Cl.: A61B 17/72

(54) **Tubular intramedullary nail for reducing long bone fractures**
Röhrenförmiger Marknagel zum Stabilisieren von Röhrenknochenbrüchen
Broche intramédullaire tubulaire pour réduire les grandes fractures osseuses

(30) Priority: 24.05.2006 IT BO20060396
(43) Date of publication of application: 28.11.2007
(73) Proprietor: CITIEFFE S.r.l., 40012 Calderara di Reno (Bologna) (IT)
(72) Inventor: Mingozzi, Franco, 40012, CALDERARA DI RENO BO (IT); Dovesi, Alan, 40100, BOLOGNA (IT)
(74) Representative: Modiano, Micaela Nadia

(56) References cited:
- WO-A-2005/009263
- US-A1- 2001 053 912

## Description

The present invention relates to a tubular intramedullary nail for reducing fractures of long bones, particularly the humerus.

Tubular intramedullary nails generally comprise a tubular stem which, in order to allow easy insertion in the medullary canal and avoid bone traumas, is appropriately curved and gradually tapers from the proximal end toward the distal end, i.e., the end inserted in the medullary canal. The stem, in the regions proximate to the proximal and distal ends, is provided with a plurality of diametrically transverse holes which are angled differently with respect to the axis of the stem and through which through screws are driven; some of said screws are designed to fix the stem to the interior of the medullary canal and others are designed to retain the parts of the bone involved in the fracture. This is the case, for example, of the fracture of the head of the humerus, which is set by inserting a nail in the proximal end of the humerus and rigidly coupling the head to the humerus by means of screws which, after being driven through holes in the nail, engage in the fractured head, so as to keep its fracture plane in contact with, and pressed against, the fracture plane of the humerus.

The stability of the fixation of the nail in the intramedullary canal and of the screws in the bone depends substantially on how firmly the screws are implanted in the bone. Moreover, when the bone is affected by connective tissue deficiency, as occurs for example in patients with osteoporosis, the screws tend to loosen, compromising the reduction of the fracture.

WO-A-2005/009263 discloses an intramedullary nail having a transversal borehole for a locking screw, and a pin or stud component that narrows the cross-sectional profile of the borehole while still permitting passage of the locking screw.

The aim of the present invention is to provide a tubular intramedullary nail which does not require substantial changes to the surgical procedure required to reduce the fracture and in particular simplifies its execution and allows to deal with several surgical situations.

Within this aim, an object of the present invention is to provide a tubular intramedullary nail which prevents the screws from loosening, so as to ensure perfect course of the fracture healing process.

Another object of the present invention is to provide a tubular intramedullary nail which does not entail appreciable changes of the steps of the operating procedure.

Another object of the present invention is to provide a tubular intramedullary nail which can be performed with a technical solution which is simple and therefore very low in cost.

In accordance with the invention, there is provided an intramedullary nail for reducing long bone fractures, as defined in the appended claims.

Further characteristics and advantages of the present invention will become better apparent from the following detailed description on the basis of the accompanying drawings, which illustrate some embodiments shown merely by way of nonlimiting example. In the accompanying drawings:
Figure 1 is a view of an intramedullary nail crossed by the screws for fixing to the bone, according to the present invention;
Figure 2 is a top view of the nail of Figure 1;
Figure 3 is an axial view of the nail of Figure 1;
Figure 4 is a sectional view, taken along the line IV-IV of Figure 2;
Figure 5 is a sectional view, taken along the line V-V of Figure 1;
Figure 6 is a sectional view, taken along the line V-V of Figure 1 but without the fixing screws;
Figure 7 is a partial view of an intramedullary nail, related to a second embodiment of the invention;
Figure 8 is a sectional view, taken along the line VIII-VIII of Figure 7;
Figure 9 is a sectional view, taken along the line VIII-VIII of Figure 7, but without the fixing screws;
Figure 10 is a partial sectional view of a third embodiment of the invention;
Figure 11 is a view of the filament used in the nail of Figure 10;
Figure 12 is a sectional view, taken along the line XII-XII of Figure 10;
Figure 13 is a view of another embodiment of the filament.

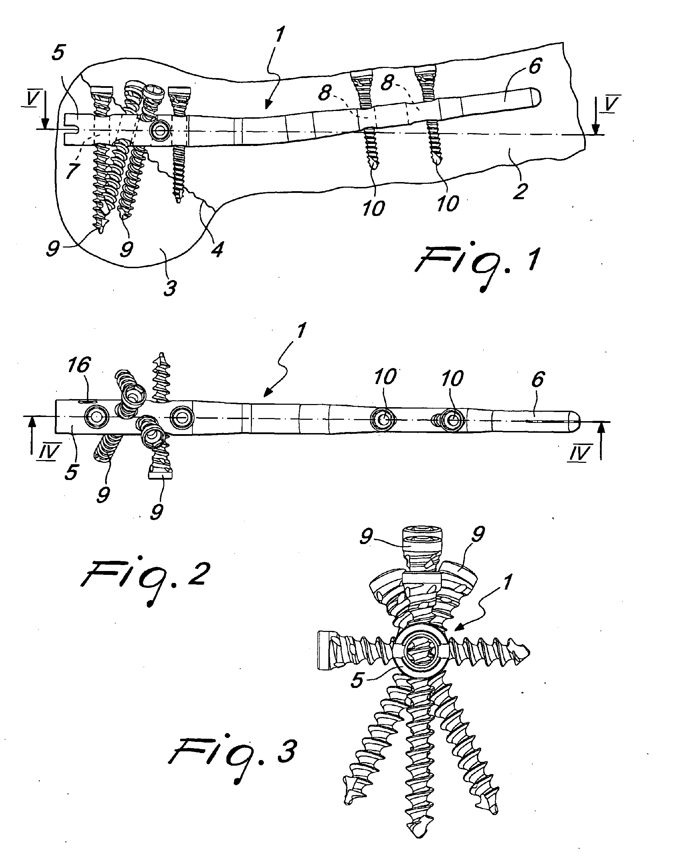

With reference to Figures 1 to 6, the reference numeral 1 designates an intramedullary nail according to the invention for reducing the fracture of a long bone, typically the femur or humerus. Figure 1 shows, merely by way of example, in broken lines the part of a humerus 2 whose spherical head 3 for articulation in the glenoid cavity of the scapula is affected by a fracture fissure 4, whose plane lies transversely to the longitudinal axis of the bone.

The nail 1 is constituted by a tubular stem, in which there is a proximal portion 5 and a distal portion 6. The proximal portion 5 is wider than the distal portion 6, which moreover, differently from the proximal portion, tapers at the end. Moreover, the stem 1 is slightly angled in order to follow the curved shape of the medullary canal of the bone 2 in which it is to be inserted.

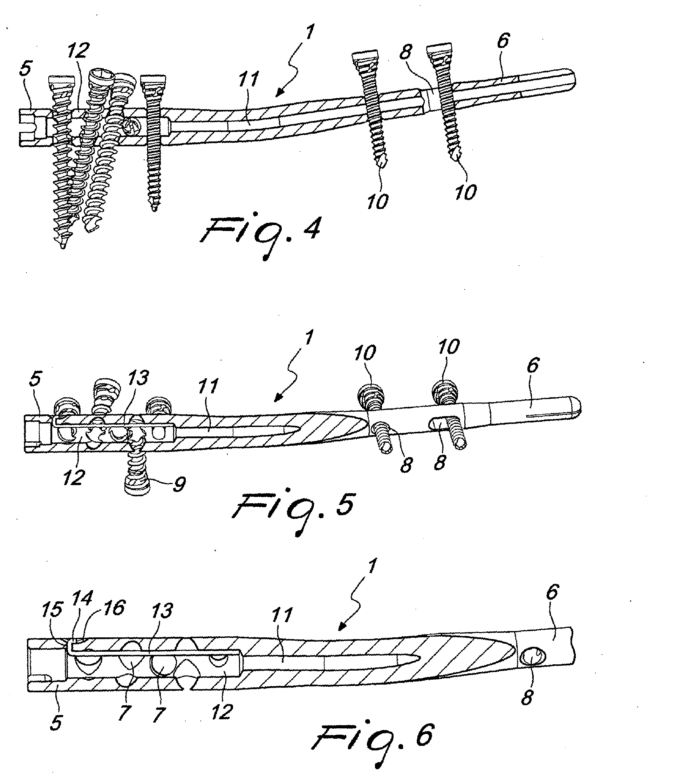

A plurality of holes 7 and 8 respectively are formed through the proximal portion 5 and the distal portion 6 of the stem 1 and, as shown more clearly in Figure 6, are distributed axially and are arranged on radial planes which are mutually angularly offset (see Figure 3). The holes 7, 8 are crossed by respective screws 9, 10, which are designed to fix the fractured bone fragments and lock the stem 1 within the bone. Conveniently, the holes 7 of the proximal portion 5 are perpendicular or inclined (see Figure 1) with respect to the axis of the stem, in order to allow the screws 9 that pass through them to engage through the fracture plane and keep together as effectively as possible the fragments of the bone to be joined.

Likewise, the holes 8 of the distal portion 6 (see Figures 2 and 5) are slotted, in order to allow the screws 10 to be oriented in the most appropriate manner to ensure the firmest coupling of the stem in the medullary canal. The screws 9, 10 have the same diameter as the respective holes 7, 8, so that they can slide freely axially within the holes 7, 8.

The stem 1 is crossed by an axial cylindrical channel 11 (see Figure 4), which in the proximal portion 5 and along its length widens and forms a cylindrical seat 12 which crosses all the holes 7. The diameter of the seat 12 is slightly larger than the diameter of the holes 7, so that the thread of the screws 9 can be considered to be internally tangent with respect to the seat 12 and the holes 7.

The seat 12 accommodates a filament 13 (see Figures 5, 6), which has an end portion 14 which is folded at right angles and is inserted within a radial hole 15, which leads to a slot 16 formed outside the proximal portion 5. The slot 16 runs parallel to the axis of the stem 1 in order to accommodate a final segment of the portion 14, which is folded in a hook-like configuration in order to allow the filament to remain fixed and positioned in the seat 12.

The filament 13 runs axially within the seat 12, at the openings of the holes 7, so that it can be diverted by the fixing screws 9 driven through said holes. Further, the filament 13 has a such a diameter as to be jammed between the internal wall of the seat 12 and the threads of the screws 9. Conveniently, the jamming is such that it produces a locking effect on the screws 9 which is substantially due to the friction with which the filament remains clamped between the turns of the threads and is increased by the undulated shape that the filament assumes due to the deflections imparted by the screws when they are screwed through the holes.

As can be seen, the considerable advantage offered by the described nail is that the screws, with respect to the background art, are locked both rotationally and axially within the nail 1, so that they cannot loosen and unscrew from the bone parts and fragments through which they are driven.

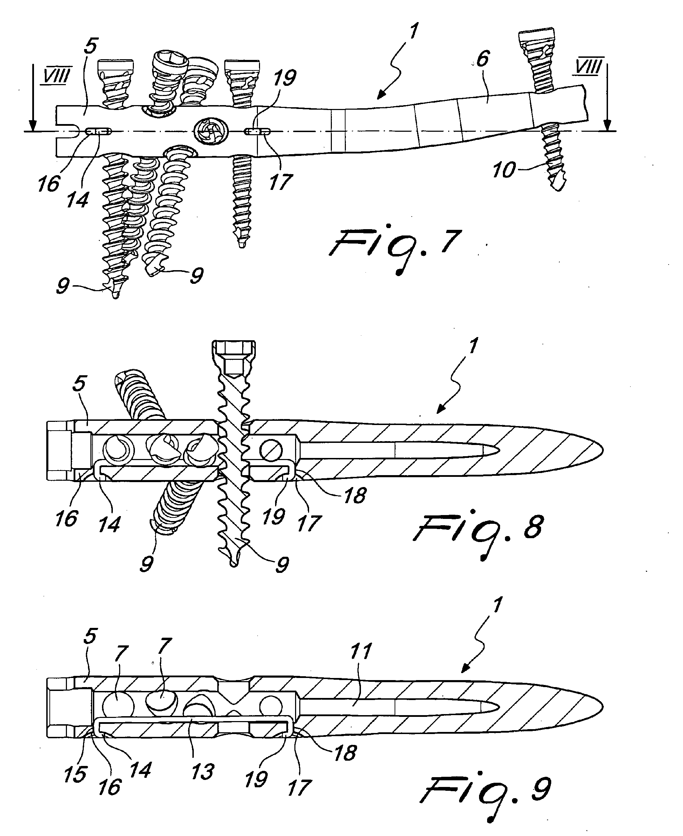

Numerous modifications and variations are possible in the practical embodiment of the invention and all are within the scope of the appended claims. Figures 7, 8 and 9 illustrate an embodiment in which the same elements, or parts of the embodiment described above, are designated by the same reference numerals. The embodiment of Figures 7, 8, 9 differs, however, in that an additional slot 17 is formed in the portion 5 and is connected to the seat 12 by a respective hole 18 and is adapted to receive the end portion 19, which is optionally curved in a hook-like shape, of the end of the filament 13 that lies opposite the portion 14.

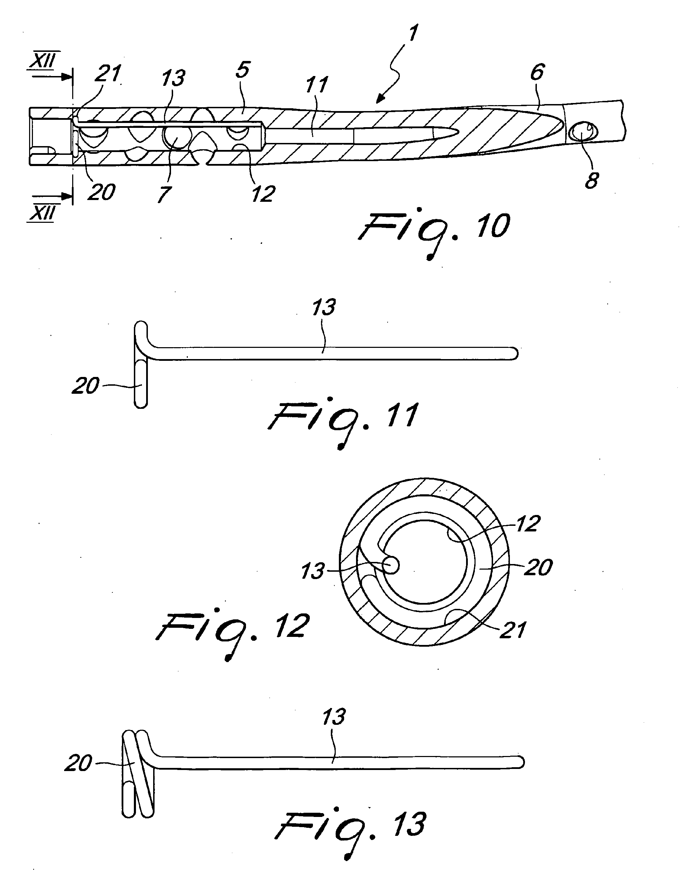

In another embodiment of the invention, it is possible to adopt a plurality of filaments, particularly two filaments, within the seat 12. Of course, the number of filaments, their diameter and the shape of their cross-section are selected according to the type of screws used and to the dimensions of the nail. Figures 10-12 illustrate another embodiment of the invention, which consists of the filament 13 in which an end portion is folded so as to form a tangent elastic ring 20 which lies on a plane which is perpendicular thereto. The ring 20 is sized so as to engage by expansion in an annular groove 21, which is formed within the seat 12, while the filament 13 lies axially on the surface of the seat 12, as shown more clearly in Figure 12.

Figure 13 is a view of another embodiment in which the ring 20 is composed of a pair of turns instead of a single turn.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. An intramedullary nail for reducing long bone fractures, comprising a tubular stem (1) which has, in the proximal end portion (5), diametrically aligned holes (7) for the passage of nail fixing screws (9), said holes (7) being distributed axially at said end portion (5) and being arranged on radial planes which are mutually angularly offset, the nail further comprising at least one filament (13) arranged inside a cylindrical seat (12) of said tubular stem (1) at said end portion (5), which runs axially and has such a cross-section that when the screws (9) are driven through said holes (7) to fix the nail in the bone, said at least one filament (13) assumes an undulated shape due to deflections imparted by said screws (9) and remains jammed between the internal wall of said stem (1) and the thread of said screws (9), so as to lock said screws (9) within the nail, said filament (13) further comprising a folded or curved end portion (14;19;20) engaging against said tubular stem (1) to fix said filament (13) inside said seat (12).

2. The nail according to claim 1, **characterized in that** said stem (1) is crossed by an axial cylindrical channel (11), which widens in said proximal portion (5) and along the length of said portion, forming said cylindrical seat (12) which passes through all the holes (7), said seat (12) having a slightly larger diameter than said holes (7), so that the thread of the screws (9) is substantially tangent inside said seat (12) and said holes (7).

3. The nail according to one of claims 1 and 2, **characterized in that** said filament (13) runs axially within said seat (12) at the openings of said holes (7), so that it can be diverted by the fixing screws (9) driven through them.

4. The nail according to one of claims 1 and 3, **characterized in that** said filament (13) has such a diameter that it can be jammed between the internal wall of said seat (12) and the threads of said screws (9).

5. The nail according to claim 1, **characterized in that** said filament (13) has a right-angled end portion (14) which is inserted in a radial hole (15) formed in said proximal portion (5).

6. The nail according to claim 5, **characterized in that** said hole (15) leads into a slot (16) which is formed outside said proximal portion (5) and lies parallel to the stem (1) in order to accommodate a final segment, which is bent so as to be hook-shaped, of said end portion (14).

7. The nail according to one of claims 5 and 6, **characterized in that** an additional slot (17) is formed in said proximal portion (5) and is connected to said seat (12) by a respective radial hole (18) and is adapted to receive the end portion (19) of said filament (13) which lies opposite said first portion (14).

8. The nail according to claim 1, **characterized in that** said filament (13) has an end portion which is bent so as to form an elastic ring (20) which is tangent and arranged on a plane which is perpendicular thereto and is adapted to engage by expansion in an annular groove (21) which is formed within said seat (12).

9. The nail according to claim 8, **characterized in that** said ring (20) is formed by a plurality of turns.

10. The nail according to one of the preceding claims, **characterized in that** two filaments (13) are accommodated within said seat (12).

## Patentansprüche

1. Marknagel zum Stabilisieren von Röhrenknochenbrüchen, der einen röhrenförmigen Schaft (1) aufweist, welcher in dem proximalen Endabschnitt (5) diametral ausgefluchtete Bohrungen (7) für den Durchgang von den Nagel befestigenden Schrauben (9) hat, wobei die Bohrungen (7) an dem Endabschnitt (5) axial verteilt sind und in radialen Ebenen angeordnet sind, die im Winkel zueinander versetzt sind, wobei der Nagel ferner mindestens ein im Inneren eines zylindrischen Sitzes (12) des röhrenförmigen Schafts (1) an dem Endabschnitt (5) angeordnetes Drahtstück (13) aufweist, das axial verläuft und einen derartigen Querschnitt hat, dass, wenn die Schrauben (9) durch die Bohrungen (7) geschraubt werden, um den Nagel in dem Knochen zu befestigen, das mindestens eine Drahtstück (13) aufgrund von durch die Schrauben (9) herbeigeführten Biegungen eine gewellte Form annimmt und zwischen der Innenwand des Schafts (1) und dem Gewinde der Schrauben (9) eingeklemmt bleibt, um die Schrauben (9) innerhalb des Nagels zu arretieren, wobei das Drahtstück (13) ferner einen gefalteten oder gebogenen Endabschnitt (14; 19; 20) aufweist, der an dem röhrenförmigen Schaft (1) anliegt, um das Drahtstück (13) im Inneren des Sitzes (12) zu befestigen.

2. Nagel nach Anspruch 1, **dadurch gekennzeichnet, dass** der Schaft (1) von einem axialen zylindrischen Kanal (11) durchgriffen wird, der in dem proximalen Abschnitt (5) und entlang der Länge dieses Abschnitts breiter wird und den zylindrischen Sitz (12) bildet, welcher durch alle der Bohrungen (7) hindurchgeht, wobei der Sitz (12) einen geringfügig größeren Durchmesser als die Bohrungen (7) hat, so dass das Gewinde der Schrauben (9) im Wesentlichen das Innere des Sitzes (12) und der Bohrungen (7) berührt.

3. Nagel nach einem der Ansprüche 1 und 2, **dadurch gekennzeichnet, dass** das Drahtstück (13) innerhalb des Sitzes (12) an den Öffnungen der Bohrungen (7) axial verläuft, so dass es mittels der durch diese hindurch geschraubten Befestigungsschrauben (9) abgelenkt werden kann.

4. Nagel nach einem der Ansprüche 1 und 3, **dadurch gekennzeichnet, dass** das Drahtstück (13) einen solchen Durchmesser hat, dass es zwischen der Innenwand des Sitzes (12) und den Gewinden der Schrauben (9) einklemmbar ist.

5. Nagel nach Anspruch 1, **dadurch gekennzeichnet, dass** das Drahtstück (13) einen rechtwinkligen Endabschnitt (14) hat, der in eine radiale Bohrung (15) eingesetzt ist, die in dem proximalen Abschnitt (5) ausgebildet ist.

6. Nagel nach Anspruch 5, **dadurch gekennzeichnet, dass** die Bohrung (15) in einen Schlitz (16) führt, der außerhalb des proximalen Abschnitts (5) ausgebildet ist und parallel zu dem Schaft (1) liegt, um ein Endsegment des Endabschnitts (14) aufzunehmen, welches hakenförmig gebogen ist.

7. Nagel nach einem der Ansprüche 5 und 6, **dadurch gekennzeichnet, dass** ein zusätzlicher Schlitz (17) in dem proximalen Abschnitt (5) ausgebildet ist und mit dem Sitz (12) durch eine entsprechende radiale Bohrung (18) verbunden ist und dazu angepasst ist, den Endabschnitt (19) des Drahtstücks (13) aufzunehmen, der dem ersten Abschnitt (14) gegenüber liegt.

8. Nagel nach Anspruch 1, **dadurch gekennzeichnet, dass** das Drahtstück (13) einen Endabschnitt hat, der derart gebogen ist, dass er einen elastischen Ring (20) bildet, welcher eine Ebene berührt und in dieser angeordnet ist, die senkrecht zu diesem liegt, und dazu angepasst ist, durch Aufweitung mit einer ringförmigen Nut (21) in Eingriff zu gelangen, welche innerhalb des Sitzes (12) ausgebildet ist.

9. Nagel nach Anspruch 8, **dadurch gekennzeichnet, dass** der Ring (20) durch eine Mehrzahl von Wicklungen gebildet ist.

10. Nagel nach einem der vorhergehenden Ansprüche, **dadurch gekennzeichnet, dass** zwei Drahtstücke (13) innerhalb des Sitzes (12) aufgenommen sind.

## Revendications

1. Broche intramédullaire pour réduire les fractures d'os longs, comprenant une tige tubulaire (1) qui a, dans la partie d'extrémité proximale (5), des trous (7) diamétralement alignés pour le passage des vis de fixation de broche (9), lesdits trous (7) étant distribués axialement sur ladite partie d'extrémité (5) et étant ménagés dans des plans radiaux qui sont mutuellement décalés d'un certain angle, la broche comprenant en outre au moins un filament (13) aménagé à l'intérieur d'un siège cylindrique (12) de ladite tige tubulaire (1) dans ladite partie d'extrémité (5), qui s'étend axialement et a une coupe transversale telle que, lorsque les vis (9) sont enfoncées à travers lesdits trous (7) pour fixer la broche dans l'os, ledit au moins un filament (13) adopte une forme ondulée due à des déviations appliquées par lesdites vis (9) et reste coincé entre la paroi interne de ladite tige (1) et le filet desdites vis (9), de manière à verrouiller lesdites vis (9) à l'intérieur de la broche, ledit filament (13) comprenant en outre une partie d'extrémité pliée ou incurvée (14 ; 19 ; 20) s'engageant contre ladite tige tubulaire (1) pour fixer ledit filament (13) à l'intérieur dudit siège (12).

2. Broche selon la revendication 1, **caractérisée en ce que** ladite tige (1) est traversée par un canal cylindrique axial (11), qui s'élargit dans ladite partie proximale (5) et sur la longueur de ladite partie, formant ledit siège cylindrique (12) qui passe à travers tous les trous (7), ledit siège (12) ayant un diamètre légèrement plus grand que celui desdits trous (7), de sorte que le filet des vis (9) soit sensiblement tangent à l'intérieur dudit siège (12) et desdits trous (7).

3. Broche selon l'une quelconque des revendications 1 et 2, **caractérisée en ce que** ledit filament (13) s'étend axialement à l'intérieur dudit siège (12) dans les ouvertures desdits trous (7), de sorte qu'il puisse être dévié par les vis de fixation (9) enfoncées à travers ceux-ci.

4. Broche selon l'une quelconque des revendications 1 et 3, **caractérisée en ce que** ledit filament (13) a un diamètre tel qu'il puisse être coincé entre la paroi interne dudit siège (12) et les filets desdites vis (9).

5. Broche selon la revendication 1, **caractérisée en ce que** ledit filament (13) a une partie d'extrémité à angle droit (14) qui est insérée dans un trou radial (15) formé dans ladite partie proximale (5).

6. Broche selon la revendication 5, **caractérisée en ce que** ledit trou (15) mène dans une fente (16) qui est formée à l'extérieur de ladite partie proximale (5) et s'étend parallèlement à la tige (1) afin de loger un segment final, qui est recourbé pour prendre la forme d'un crochet, de ladite partie d'extrémité (14).

7. Broche selon l'une quelconque des revendications 5 et 6, **caractérisée en ce qu'**une fente additionnelle (17) est formée dans ladite partie proximale (5), est raccordée audit siège (12) par un trou radial respectif (18) et est adaptée pour recevoir la partie d'extrémité (19) dudit filament (13) qui s'étend à l'opposé de ladite première partie (14).

8. Broche selon la revendication 1, **caractérisée en ce que** ledit filament (13) a une partie d'extrémité qui est recourbée afin de former un anneau élastique (20) qui est tangent et aménagé dans un plan qui lui est perpendiculaire et est adapté pour s'engager par expansion dans une rainure annulaire (21) qui est formée à l'intérieur dudit siège (12).

9. Broche selon la revendication 8, **caractérisée en ce que** ledit anneau (20) est formé d'une pluralité de spires.

10. Broche selon l'une quelconque des revendications précédentes, **caractérisée en ce que** deux filaments (13) sont logés dans ledit siège (12).
